# EUROPEAN PATENT SPECIFICATION

(11) **EP 4 051 251 B1**
(45) Date of publication and mention of the grant of the patent: **23.08.2023**
(21) Application number: 21783523.0
(22) Date of filing: 29.09.2021
(51) Int. Cl.: A61K 9/08, A61K 47/02, A61K 31/137, A61P 9/02

(54) **STABLE, INJECTABLE NORADRENALINE SOLUTIONS FREE OF ANTIOXIDANTS**
STABILE INJIZIERBARE NORADRENALINLÖSUNGEN, DIE FREI VON ANTIOXIDANTIEN SIND
SOLUTIONS INJECTABLES STABLES DE NORADRÉNALINE EXEMPTES D'ANTIOXYDANTS

(30) Priority: 21.10.2020 EP 20203036
(43) Date of publication of application: 07.09.2022
(73) Proprietor: Sintetica S.A., 6850 Mendrisio (CH)
(72) Inventor: CARONZOLO, Nicola, 6850 Mendrisio (CH); DONATI, Elisabetta, 6850 Mendrisio (CH); BIANCHI, Clara, 6850 Mendrisio (CH)
(74) Representative: Hoefer & Partner Patentanwälte mbB
(86) International application number: PCT/EP2021/076849
(87) International publication number: WO 2022/083993

(56) References cited:
- US-A1- 2005 070 613
- US-A1- 2017 049 720
- US-B2- 10 653 646

## Description

### BACKGROUND OF THE INVENTION

The present invention is within the technical field of stable noradrenaline solutions free of chelating agents and antioxidants which are highly stable. In particular, it relates to such stable, injectable noradrenaline solutions, having low counts of subvisible particles. The solutions are in particular highly stable even upon prolonged storage. The present invention also pertains to processes for producing such stable noradrenaline solutions free of chelating agents and antioxidants, and uses.

Noradrenaline (N-desmethylepinephrine) (a/k/a norepinephrine) is a potent α-sympathomimetic drug and physiological neurotransmitter with the typical core structure of the phenylalkylamines (2-phenyl-1-aminoethane). The hydrogen tartrate and hydrochloride salts are widely used in injectable solutions for acute treatment of hypotension, anaphylactic shocks, and as vasoconstrictive additives in local anaesthetic formulations to prolong the analgesic effect. Noradrenaline is commonly administered in the form of solution, by intravenous slow infusion, and finds application in the case of cardiovascular collapse, in states of shock associated with low peripheral resistance, the so-called septic shock, and to restore and maintain physiological blood pressure levels.

Different categories of compositions or solutions of noradrenaline have to be distinguished in the art, each having their own and different technical requirements and background.

Conventional high concentration noradrenaline solutions, e.g. packaged in vials or ampules, are diluted in situ at the hospital into the infusion bag/solution for injection. These diluted, low concentration noradrenaline solutions are then for immediate use by injection or infusion, having limited stability and shelf life. Also, there are (low concentration) noradrenaline solutions which contain antioxidants and/or chelating agents (or other preservatives) to allow stabilizing them. WO 2018/140894 A1 and US 2016/0058715 A1 disclose high volume ready to use bags of noradrenaline bitartrate and propose EDTA as a chelating agent and oxygen scavenger.

The technical requirements of the aforementioned noradrenaline solutions are totally different from the present field of stable noradrenaline solutions free of antioxidants and/or chelating agents.

In contrast, the present invention addresses the problem of providing a highly stable noradrenaline solution free of chelating agents, antioxidants (such as sulphites) and preserving agents. It also addresses the problem of finding a method for producing such a noradrenaline solution free of chelating agents, antioxidants (such as sulphites) and preserving agents under conditions which promote and ensure a high stability level upon storage, and defining the key thresholds of oxygen using inert gas during the production method.

US 2005/0070613 A1 in this regard discloses a method for producing, free of chelating agents and antioxidants, stabilized solutions based on phenolic substances which consists in deoxygenation of the solutions with an inert gas, and in deoxygenation of gas holdups of the vessels, of the manufacturing pipes and inerting of ampoules and flasks containing the solution with a dense inert rare gas such as argon, at low temperature and with pH adjusted above 3.0 and below 5.0 to obtain stable solutions of phenolic substances containing not more than 0.02 ppm of oxygen in the solution, which is filtered by double sterilizing filtration useful for human or animal therapeutic treatment. Regarding noradrenaline solutions and their production, US2005/0070613 A1 discloses in Example 1 "perfect" control of dissolved oxygen along the manufacturing steps. The dissolved oxygen in the finished products has a specification of ≤ 3 ppm. Mean values of 0.98 or 0.83 ppm, respectively are disclosed (see paragraph [0105]). No specific oxygen threshold for the headspace of the containers is specified in US2005/0070613 A1. The argon, nitrogen and oxygen content was analysed (see paragraph [0108]) and the oxygen content in the headspace was found to be between 3.0 and 1.8 % v/v. Some of the values for the composition of the headspace do by far not add up to 100% and are obviously erroneous.

U.S. Patent No. 10,251,848 discusses the relevance of pH to the stability of noradrenaline, in addition to the need to limit dissolved oxygen concentrations in the finished product. No specification for the headspace of the finished product is disclosed.

US2017/049720A relates to a process for producing a stable, injectable solution with low content of noradrenaline, which includes dissolving noradrenaline and optionally an excipient in deoxygenated or degassed water, filtrating the resulting noradrenaline solution in a nitrogen current, distributing the solution in a nitrogen current, and sterilization, preferably hot. It further discloses a stable, injectable solution with low content of noradrenaline, substantially free of anti-oxidizing and preservative agents, as well as uses thereof in the medical and pharmaceutical fields.

While mechanisms of noradrenaline degradation have received much attention over the years, very little attention has been given to subvisible particles, and mechanisms for their control. This is in spite of the required standards, such as United States Pharmacopoeia (USP), which imposes limits on subvisible particles in injectable solutions in chapter 788.

Traun et al., International Journal of Pharmacy Compounding, Vol. 10 No. 3, May/June 2006, reports that subvisible particles in sterile injectable products can originate from many sources, including the solution itself and its ingredients, the production process and its variables (e.g., environment, equipment, personnel), the product's packaging, and the preparation of the product for administration (e.g., manipulating the product, the environment in which it is prepared). However, very little has been published on the formation of subvisible particles by solutions of noradrenaline.

What is needed are new methods and formulations for controlling the stability of noradrenaline injectable drug products/solutions free of antioxidants (such as sulphite(s) or ascorbic acid) and/or chelating agents (such as EDTA, EGTA, DTPA and the like), particularly for controlling the generation of subvisible particles in such (low concentration) noradrenaline solutions.

### SUMMARY OF THE INVENTION

During the development of the different aspects of the present invention, the inventors have made several findings important to the reduction of subvisible particles, and the stability of noradrenaline formulations. For example, it was surprisingly found that the formation of subvisible particles can be controlled by limiting the amount of oxygen in the headspace to levels where no oxygen degradation can be observed. It was also unexpectedly found that both the oxygen content in the headspace as well as the dissolved oxygen content in the solution have to be tightly controlled within the limits disclosed herein, in order to ensure the low numbers of subvisible particles during storage. While subvisible particles in (dilute) noradrenaline solutions do not appear to result from oxidation, as their formation occurs at concentrations below which oxidative degradation is observed, the concentration of oxygen in the headspace does affect their formation. Consequently, the production of subvisible particles can be controlled by limiting the amount of oxygen in the headspace of the drug product to levels below which no oxidative degradation can be observed.

According to a first aspect, the present invention provides a process for producing a solution containing noradrenaline, free of antioxidants (such as sulfite(s) or ascorbic acid) and in particular also free of chelating agents, comprising the following steps:
a. dissolving noradrenaline and optionally one or more excipients in deoxygenated or degassed water, in order to produce a solution containing noradrenaline;
b. distributing the noradrenaline solution in an inert gas current into a container,
c. optionally sterilizing the solution containing noradrenaline, before step b) and/or after step d),
d. hermetically sealing the container,
e. characterized in that the oxygen concentration in the solution containing noradrenaline is less than 300 ppbw immediately after sealing the container; and the oxygen concentration in the headspace, in particular immediately after sealing the container, is at most 1.0 percent v/v.

Thus, it has been surprisingly found that a (gaseous) headspace above/in contact with a noradrenaline solution, the headspace having an oxygen concentration of at most 1.0 percent v/v, preferably less than 1.0 percent v/v, more preferably 0.95 % v/v or less, even more preferably 0.9 % v/v or less, even more preferably 0.8 % v/v or less, allows significantly reducing the formation of subvisible particles in the noradrenaline solution.

According to a preferred embodiment, the aforementioned oxygen content in the headspace should be present in the container immediately after sealing the container. Both disruptive and non-disruptive methods for determining the oxygen content in the headspace and solution are known in the art, including pharmacopoeia. One non-invasive method is Frequency Modulation Spectroscopy (FMS), where light from a near-infrared semiconductor laser is tuned to match the internal vibrational frequency of the oxygen molecule in the container (see e.g. https://www.lighthouseinstruments.com/uploads/New_documents_9Jan2015/LIGHTHOUSE_ Oxygen_Monitoring_AppNote102_2015_PRINT.pdf), although other methods exist including electrochemical methods. An invasive draw, where the container is physically breached and a sample is taken from the headspace and/or solution is another way. Another way is to calculate the content based on other oxygen values. Thus, for example, following the procedure in Example 2, it is possible to calculate the concentration of the oxygen in the headspace immediately after the container is sealed knowing the oxygen content in the solution immediately after the container is filled, the oxygen content in the solution or headspace at equilibrium, the volume of the headspace, and the total volume of the container. In one example, a 3600 Series Analyzer for Oxygen (Orbisphere, CH) can be used according to the manufacturer's instructions. See also e.g. J. Pharm. Biomed. Anal., 2008 Sep 10; 48(1): 8-12; "A novel GC-MS method for rapid determination of headspace oxygen in vials of pharmaceutical formulations" L. Wu et al.

It was surprisingly found that a very limited and specific oxygen content of the headspace in the container (together with a specific content of dissolved oxygen in the solution in the container) is important to prevent or minimize formation of subvisible particles. It is assumed - without being bound to this theory - that after (filling and) sealing the container, also before equilibrium conditions can be reached, the contact area between the noradrenaline solution and the headspace is of great importance regarding the question whether or to which extent subvisible particles are formed. At this contact area, the oxygen in the headspace meets the noradrenaline in the solution and different interactions and reactions can occur depending on the concentration of oxygen in the headspace.

Surprisingly, this was found even at a level of oxygen which does not appear to be decisive regarding the oxidative degradation of noradrenaline, such as monitored by the formation of arterenone as the oxidative degradation product of noradrenaline.

Noradrenaline is preferably used as its (R)-isomer. In particular, it is enantiomerically substantially pure, i.e. at least 98% by weight of the total noradrenaline present is (R)-Noradrenaline.

Also, according to a further preferred embodiment of the invention, it was found that the formation of subvisible particles is even lower if the oxygen concentration in the headspace immediately after sealing the container with the noradrenaline solution is within a certain low range of about 0.2 to about 1.0 percent v/v, preferably in the range of about 0.3 to about 0.95 percent v/v, in particular about 0.5 to about 0.95 percent v/v. Thus, it was observed that an extremely low oxygen concentration close to zero in the headspace leads to the formation of more subvisible particles compared to a slightly higher oxygen concentration within the aforementioned ranges. It is assumed that such extremely low oxygen concentrations close to zero lead to different interactions and reactions of the noradrenaline which increase formation of subvisible particles.

It was further found that the conditions for the formation of subvisible particles are also influenced by the oxygen concentration in the solution containing noradrenaline which comes into contact with the headspace. Thus, the concentration of dissolved oxygen in the noradrenaline solution should be less than 300 ppbw, preferably less than 100 ppbw immediately after sealing the container. Again, it is assumed that a low concentration of dissolved oxygen in the noradrenaline solution influences the conditions leading to the formation of subvisible particles at the surface/contact area with the headspace.

Within the present invention, noradrenaline, also called norepinephrine, is understood to encompass noradrenaline free base as well as all pharmaceutically acceptable salts, or mixtures thereof. Preferably, but not limiting, it encompasses the bitartrate (hydrogentartrate) or hydrochloric salts of noradrenaline, in particular the bitartrate salt.

Within the present invention, the terms "noradrenaline solution" and "solution containing noradrenaline" are used synonymously, and encompass a solution, preferably an aqueous solution of noradrenaline or a pharmaceutically acceptable salt thereof.

Within the present invention, "injectable" means suitable to be injected into a patient (human or animal). Typically, the noradrenaline solution of the invention is administered by intravenous or intra-arterial injection. In certain embodiments, there is provided an infusion of a therapeutically active amount of the noradrenaline solution according to the invention.

Regarding the absence of antioxidants, within a preferred aspect of the present invention, the term "antioxidant" shall mean any sulphur containing and/or sulfite antioxidants such as sodium sulfite, sodium bisulfite, sodium metabisulfite or mixtures thereof. In a more preferred embodiment, "antioxidant" additionally includes any of the group of non-sulfite antioxidants including butylated hydroxylanisol, ascorbic acid, propyl gallate, vitamin E, alpha-tocopherol, butylated hydroxyl toluene or ascorbic acid, or mixtures thereof. According to one embodiment of the present invention, when a formulation is said to lack antioxidants in this document, it will be understood that the formulation could be defined as lacking any antioxidant as that term is traditionally used in the art, as described, for example, in Rowe et al., Handbook of Pharmaceutical Excipients, 6th Edition 2009.

Regarding the absence of chelating agents, within a preferred aspect of the present invention, the term "chelating agent(s)" shall mean a metal ion chelator selected from the group consisting of EDTA (edetic acid), EGTA, and diethylenetriaminepentaacetic acid, or mixtures thereof, in particular at concentrations in the solution at a concentration of between about 1 and 100 ug/ml. In a more preferred aspect of the present invention, "chelating agent" od "chelator" includes, tricarboxylic acids, and aminopolycarboxylic acids such as ethylenediaminetetraacetic acid (EDTA), ethylene glycol-bis(3-aminoethyl ether)-N,N,N',N'-tetraacetic acid (EGTA), and penta(carboxymethyl)diethylenetriamine (or diethylenetriaminepentaacetic acid; DTPA), and salts and hydrates thereof, and optionally various dicarboxylic acids.

According to one embodiment of the present invention, the nordadrenaline solutions as described herein are free of any chelating agent known in the art which is added to a solution of noradrenaline of a pharmaceutically acceptable salt thereof in order to protect noradrenaline from degradation. According to one embodiment of the present invention, when a formulation is said to lack chelating agents in this document, it will be understood that the formulation could be defined as lacking any chelating agent as that term is traditionally used in the art, as described, for example, in Rowe et al., Handbook of Pharmaceutical Excipients, 6th Edition 2009. Examples of chelating agents include in particular metal ion chelators, such as EDTA, EGTA, DTPA and the like.

In a particularly preferred embodiment of the present invention, "free of antioxidants" means free of sulfite(s), including bisulfite(s) and metabisulfite(s), and, preferably, "free of chelating agents" means free of EDTA (including edetic acid or any salt thereof such as disodium edetate), EGTA and/or DTPA.

A "low concentration" or "very low concentration" noradrenaline solution, within the different aspects of the invention as disclosed herein, is a noradrenaline solution having a concentration of 0.1 mg/ml or less. According to a preferred embodiment and definition, the "low" or "very low" concentration of noradrenaline in the noradrenaline containing solution is less than 0.1 mg/ml, preferably equal or less than 0.06 mg/ml, more preferably less than 0.04 mg /ml, in particular less than 0.025 mg/ml. According to a more preferred embodiment, the concentration of noradrenaline in the "low" or "very low" concentration noradrenaline solution is in the range from 0.001 to 0.04 mg/ml.

Preferably, within the present invention, the term "stable" in relation to a noradrenaline solution means that it contains less than 0.5 %, in particular less than 0.05% by weight of arterenone, based on the total weight of noradrenaline present in the solution. According to one embodiment, the drug product can also be defined based on its stability, and the presence of impurities (i.e. by-products from the manufacturing process of noradrenaline, degradants of noradrenaline, and contaminants, but excluding residual solvents) in the solution. In preferred embodiments, the solution comprises no more than 1%, 0.5%, 0.2%, or even 0.1% (w/w) impurities and degradants of noradrenaline.

The concentrations of noradrenaline or a pharmaceutically acceptable salt thereof are generally calculated and indicated as noradrenaline free base, unless otherwise stated. Also, the concentrations of noradrenaline or a pharmaceutically acceptable salt thereof are indicated as mg or µg, calculated as noradrenaline free base, per ml of the total solution, unless indicated otherwise. The concentration of oxygen is indicated as percent (%) v/v for the headspace (based on the total volume of the headspace), or ppbw (parts per billion by weight) for the dissolved oxygen. Preferably, the headspace, apart from the oxygen concentration indicated, consists of or essentially consists of only inert gases as specified herein, or mixtures thereof. Thus, oxygen is the only gas in the headspace, which can oxidize noradrenaline. Preferably, the headspace contains at least 90 % v/v, more preferably at least 95% v/v of nitrogen and /or agon, more preferably at least about 98 or about 99% v/v. It is further preferred, that nitrogen is the only inert gas used in the headspace. More preferably, apart from oxygen as well as nitrogen and/or argon (as preferred inert gases, see above), the only gases which may be present in the headspace are those present in air, in an amount less than 0.05 % v/v, in particular less than 0.005 % v/v, apart from CO₂ even less than 1x10-4 % v/v. Thus, as the headspace is protected from oxygen (and air) during production, the traces of components of air, which may be present in the headspace, are preferably very low.

The term "subvisible particles", as used herein, means subvisible particles ≥ 10 µm and/or subvisible particles ≥ 25 µm. According to the present invention and its different aspects, the formation of subvisible particles of at least one of the aforementioned categories, i.e. subvisible particles ≥ 10 µm or subvisible particles ≥ 25 µm, is reduced. More preferred, both categories of subvisible particles, i.e. subvisible particles ≥ 10 µm and subvisible particles ≥ 25 µm, are reduced according to the present invention. These categories are commonly determined according to pharmaceutical standards. Subvisible particle counts were measured using the light obscuration particle count test prescribed by USP (United States Pharmacopoeia) chapter 788 (Official as of 1 May 2013). All testing was undertaken according to stability methods prescribed by the International Conference on Harmonization, ICH Q1A (R2) (effective 1 August 2003). According to another embodiment, also the light obscuration particle count test as described in European Pharmacopoeia 10.0, Chapter 2.9.19., pages 360 to 362, may be used.

According to a preferred embodiment, referring to all aspects of the present invention, including the process for producing a solution containing noradrenaline, the solution obtained, the drug product, the process for reducing the formation of subvisible particles, and the uses according to the invention as described herein, the subvisible particle counts ≥ 10 µm per ml of solution containing noradrenaline are less than 10, in preferably less than 5, in particular less than 3, after 3 months storage, or preferably even 6 month storage in a hermetically sealed container at 25°C and 60% relative humidity (RH). After 24 month storage in a hermetically sealed container at 25°C and 60% relative humidity (RH), the subvisible particle counts ≥ 10 µm per ml of solution containing noradrenaline are less than 50, in preferably less than 40, in particular less than 30.

Further preferred, the subvisible particle counts ≥ 25 µm per ml of solution containing noradrenaline are less than 2, in preferably 1 or less after 12 months storage, or preferably 24 months storage in a hermetically sealed container at 25°C and 60% relative humidity (RH).

In particularly preferred embodiments, the noradrenaline solution of the invention will maintain the aforementioned subvisible particle counts for 12 or even 24 months when stored at 25 °C protected from light. The drug product will preferably experience an increase in the ≥ 10 µm subvisible particle count of no more than 100%, 200%, 300%, 400%, or 500% for 12 months when stored at 25 °C and 60% relative humidity protected from light. Alternatively or in addition, the drug product will preferably experience an increase in the ≥ 25 µm subvisible particle count of no more than 100%, 200%, 300%, 400%, or 500% for 12 months when stored at 25 °C and 60% relative humidity protected from light.

Process step a) as defined above can be performed as described in WO2015/128418 A1.

Thus, in step a), typically the water used to prepare the solution is degassed or deaerated, distilled, sterile, pyrogen-free water for pharmaceutical use. According to some embodiments, the deoxygenated or degassed water is obtained by blowing or bubbling an inert gas current.

In general, for all different aspects of the present invention as disclosed herein, "inert gas" encompasses any inert gas as known in the art, such as nitrogen or argon, or other noble gases or mixtures thereof. Preferred inert gases are selected from argon and nitrogen and combinations thereof, with nitrogen being particularly preferred. Thus, according to a more preferred embodiment, the inert gas is nitrogen.

Noradrenaline and optionally any excipient(s) are dissolved in water. According to one preferred embodiment, dissolution can be carried out within a suitable inert container, in which air or oxygen have been removed by passage of inert gas. According to a further preferred embodiment, during dissolution of the noradrenaline an inert gas can be blown into the container or tank of the solution, to remove any residual oxygen. This purging with inert gas can be prolonged in order to further reduce the dissolved oxygen level in the solution. Upon completion of process step a), the noradrenaline solution obtained has a residual oxygen content (dissolved oxygen) equal to or lower than 300 ppbw, preferably equal to or lower than 100 ppbw.

In a preferred embodiment, the pH of the noradrenaline aqueous solution is adjusted to an acidic pH. According to a further preferred embodiment, the pH of the noradrenaline solution is adjusted within a range of 3.0 to 4.5. In some embodiments, the pH may be in the range of 3.2 to 3.6, more preferably within a range of 3.3 to 3.6. The pH may be adjusted by any suitable acid, for example, typically by adding HCl, e.g. 1N HCl. For increasing the pH of the noradrenaline solution, if desired, any base can be used as known in the art, such as NaOH. In step b), the noradrenaline solution is distributed in suitable containers. Any suitable container made of any suitable material known in the art for marketing noradrenaline solutions, in particular injectable noradrenaline solutions can be used. According to a preferred embodiment, the container is an ampoule or vial, more preferably a glass ampoule or vial. The containers may be depyrogenated, optionally in the presence of inert gas. Particular care is taken to minimize or avoid oxygen or air entering the container during the distributing (filling) step b). As it was found that both the oxygen content in the headspace as well as the dissolved oxygen content in the solution have to be tightly controlled within the limits disclosed herein, the parameters of the distributing devices and environments for both step b) and also step c) (see below) are optimized and verified to constantly achieve the desired oxygen levels. Extra measures may comprise increasing the flow and direct insertion of an inert gas into the container during the distribution step b), e.g. by using a coaxial needle device distributing both the noradrenaline solution and an inert gas or inert gas mixture from two coaxial flow channels into the container, or application of a drop of liquid nitrogen into the container after completely distributing (filling) the noradrenaline solution into the container, but before the container is sealed. It is also possible to place the device used for the distribution of the solution into the container in a reduced oxygen or substantially oxygen free environment for further minimizing the amount of oxygen entering the container, and to use closed filling lines excluding the ingress of oxygen.

The means as such for achieving the oxygen concentrations in the noradrenaline solution and headspace as specified in the present invention are generally known in the art, but have to be applied in a careful way, constantly monitoring the oxygen concentration in the solution and in the headspace above the noradrenaline solution in order to provide the desired oxygen levels.

According to step c), the solution is sterilized by known means. Such known means in the art comprise e.g. sterile filtration, heat treatment and/or irradiation, in particular sterile filtration and/or heat treatment. The heat treatment, if performed, may be accomplished typically at temperatures above 100°C, for a time suitable for sterilization, for example equal to or greater than 15 minutes. Preferably, the heat treatment can be a heat sterilization for 15 minutes at 121 °C, in particular in an autoclave.

The sterile filtration, if performed, may be done by passing the solution containing noradrenaline through a filter of the type for sterilization, as known in the art. Passage of the noradrenaline solution through the filter can be speeded up by blowing a current of inert gas which acts as a carrier. Suitable filters are those used in the pharmaceutical technology for preparation of sterile injectable solutions.

According to step c) the (optional) sterilisation of the solution can be performed before step b) and/or after step d) above. According to a preferred embodiment, sterilisation of the solution is performed before step b), i.e. before distributing the noradrenaline solution in an inert gas current into a container, by sterile filtration. According to another preferred embodiment of the invention, sterilisation of the solution is performed or additionally performed after step d) by heat treatment as described above.

According to a further preferred embodiment, a sterilisation of the solution is performed before step b) by sterile filtration, and then again after step d), by heat treatment.

According to step d), the container is hermetically sealed. Sealing can be done in any conventional way, taking care that during the sealing step oxygen (or air) is kept out of the container, e.g. by a flow of inert gas (such as through a coaxial needle), applying a drop of liquid nitrogen into the container before sealing, or applying vacuum or inert gas atmosphere. Similar as mentioned for step b) above, the device used for sealing the container can be placed in a reduced oxygen or substantially oxygen free environment for further minimizing the amount of oxygen entering the container during the sealing step d). Of course, a combination of these measures may be used.

Any suitable container made of any suitable material known in the art for noradrenaline solutions, in particular injectable noradrenaline solutions can be used. Depending on the kind of container used, the sealing step d) may comprise heat sealing (such as for an ampoule) or application of an airtight cap (for vials). According to a preferred embodiment, the container is an ampoule or vial, more preferably a glass ampoule or vial.

Preferably, the steps of the process of producing a noradrenaline solution as disclosed herein are carried out in sterile environments in order to avoid bacterial contamination of the noradrenaline solution.

According to a further advantageous embodiment of the invention, the volume of the headspace is less than 11 %, preferably less than 10%, more preferably less than 9%, more preferably less than 8% of the volume of the solution containing noradrenaline, and optionally the volume of the headspace is at least 1 % preferably at least 2.5 % of the volume of the solution containing noradrenaline. Thus, it was found that minimizing the volume of the headspace in relation to the volume of the noradrenaline solution in the container is a convenient way to further minimize the formation of subvisible particles and to improve the stability of the noradrenaline solution.

According to a further aspect of the present invention, the oxygen concentration in the solution containing noradrenaline in the hermetically sealed container upon equilibration at 25 °C, (60% RH) is less than 600 ppbw, preferably less than 340 ppbw. It has been found that these thresholds for the equilibrium oxygen concentration in the noradrenaline solution can surprisingly minimize the formation of subvisible particles and enhance the stability of the noradrenaline solution. The importance of the equilibrium oxygen concentration has not been recognized in the prior art, only focusing on the oxygen concentration in the noradrenaline solution when filled into the container, and not recognizing the significant impact of the change in dissolved oxygen once it is equilibrated inside the sealed container.

According to the invention, the noradrenaline is free of antioxidizing agents (antioxidants), and preferably also of chelating agents. Antioxidants are known to the skilled person in the art, and include in particular sulphite(s) or bisulfite(s) or ascorbic acid. Chelating agents are also known to the skilled person and include for example EDTA.

In a particularly preferred embodiment of the present invention, "free of antioxidants" means free of sulfite(s), including bisulfite(s) and metabisulfite(s), and, preferably, "free of chelating agents" means free of EDTA, EGTA and/or DTPA.

Preferably, it is free of antioxidants and chelating agents or other preservatives. It has been surprisingly found that the noradrenaline solutions according to the present invention are highly stable and have low counts of subvisible particles, even after prolonged storage, in the absence of antioxidants and the absence of chelating agents or other preservatives. Surprisingly, no refrigeration and no protection against light is needed.

According to a further aspect of the present invention, it has been found that if the oxygen concentration in the solution containing noradrenaline is less than 300 ppbw immediately after sealing the container; and the oxygen concentration in the headspace immediately after sealing the container is at most 1.0 percent v/v, the formation of subvisible particles can be controlled particularly well in low or very low concentration noradrenaline solutions, having in particular concentrations of 0.1 mg/ml or below, which are otherwise particularly sensitive to stability problems and oxidation. Thus, according to a preferred embodiment of the invention, the concentration of noradrenaline in the noradrenaline containing solution is less than 0.1 mg/ml, preferably equal or less than 0.06 mg/ml, more preferably less than 0.04 mg /ml, in particular less than 0.025 mg/ml. According to a more preferred embodiment, the concentration of noradrenaline in the noradrenaline solution is in the range from 0.001 to 0.04 mg/ml.

As mentioned above, according to one embodiment, a stable noradrenaline injectable solution is considered as one containing less than 0.05% by weight of arterenone. According to one embodiment, "stable" as used herein means containing (and keeping upon extended storage, e.g. 24 months at 25°C, 60%RH, or 12 months at 40°C, 75%RH) less than 0.05% by weight of arterenone, and preferable also low total impurities as specified above.

The noradrenaline solution according to the invention has a surprisingly high stability, even without refrigeration, and at high temperatures. The surprisingly low formation of subvisible particles is even observed without protection against light, and the solution remains colorless.

With the present invention, the inventors have achieved some significant advantages including a surprisingly low formation of subvisible particles and increase of stability in noradrenaline solutions, providing a significant increase in the product safety profile.

In line with the surprising findings regarding the low formation of subvisible particles in the noradrenaline solution, a further aspect of the invention is directed to a process for reducing the number of subvisible particles in a solution containing noradrenaline, wherein the solution containing noradrenaline is free of antioxidants, and wherein the solution containing noradrenaline is contained in a container, in particular a closed (or hermetically sealed) container, together with a headspace, comprising the following steps:
a. dissolving noradrenaline and optionally one or more excipients in deoxygenated or degassed water, in order to produce a solution containing noradrenaline;
b. filling the solution containing noradrenaline into a container, the filled container comprising the solution containing noradrenaline and a headspace;
c. controlling the oxygen concentration in the headspace at less than 1.0 percent v/v.

The term "controlling" in step c) as used herein includes "adjusting" and could thus be alternatively formulated as "adjusting the oxygen concentration in the headspace to be less than 1.0 percent v/v". The surprising effect of an oxygen concentration in the headspace within the above range on a low formation of subvisible particles has not been recognized or suggested in the prior art, and constitutes a significant advantage in the provision of stable noradrenaline solutions having low counts of subvisible particles. As evidence in the Examples, see in particular Table 1c in the Example section, also the early formation of subvisible particles was minimized by using a headspace with an oxygen content of less than 1% v/v immediately after sealing the vials (together with an oxygen concentration in the solution containing noradrenaline is less than 300 ppbw). Typically and preferably, the subvisible particles did not increase in the first three months of storage, if an oxygen content of less than 1% v/v oxygen in the headspace was used. Rather, the number of subvisible particles significantly decreased when comparing the counts immediately after sealing the container, and after 3 months storage. Thus, using a headspace with an oxygen content of less than 1% v/v immediately after sealing the container surprisingly even allowed for the re-dissolution of very small subvisible particles according to a preferred embodiment of the present invention.

The preferred embodiments mentioned above for the process for producing a stable solution containing noradrenaline can be equally applied to the aspect of the invention concerning the process for reducing the number of subvisible particles in a solution containing noradrenaline.

For example, the preferred oxygen concentration ranges in the headspace as well as the preferred volumes of the headspace (in relation to the noradrenaline solution), and the preferred dissolved oxygen concentration in the noradrenaline solution are as indicated above, in particular immediately after closing (sealing) the container. Also, the concentration of dissolved oxygen in the noradrenaline solution, immediately after sealing the container and upon equilibrium, and the concentration of noradrenaline in the solution are preferably as indicated above.

Details of the considered combinations of preferred embodiments are also evident from the below list of embodiments of the invention.

A further aspect of the present invention is directed to a drug product according to claims 7 to 11 comprising, or in the form of, a hermetically sealed container comprising a solution containing noradrenaline and a headspace; wherein oxygen concentration in the solution containing noradrenaline in the hermetically sealed container upon equilibration at 25 °C is less than 600 ppbw, in particular less than 340 ppbw.

The skilled person knows how to calculate the oxygen concentration upon equilibrium for a solution containing noradrenaline in a hermetically sealed container containing a headspace volume, starting from the volume and concertation of oxygen in the solution and the volume and oxygen concentration in the headspace. According to one embodiment, the oxygen concentration in the solution containing noradrenaline upon equilibration can be determined as described in Example 2. According to another embodiment of the invention, it can be determined by incubating the hermetically sealed container comprising the noradrenaline solution and the headspace at 25 °C and ambient conditions (60%RH) for 7 days, preferably 14 days.

According to another preferred embodiment of the invention, the drug product comprises (or consists of):
- a hermetically sealed container comprising a solution containing noradrenaline and a headspace,
- obtainable by a process wherein the oxygen concentration in the solution containing noradrenaline is less than 100 ppbw immediately after sealing the container; and wherein the oxygen concentration in the headspace immediately after sealing the container is less than 1.0 percent v/v.

Thus, as explained in detail above, the inventive process for producing a stable solution containing noradrenaline and a drug product as described herein surprisingly allows significantly reducing the formation of subvisible particles in the noradrenaline solution. It was found that a very limited and specific oxygen content of the headspace in the container is important to prevent or minimize formation of subvisible particles.

It is assumed - without being bound to this theory - that after (filling and) sealing the container, in particular before equilibrium conditions can be reached, the contact area between the noradrenaline solution and the headspace is of great importance regarding the question whether or to which extent subvisible particles are formed. At this contact area, the oxygen in the headspace meets the noradrenaline in the solution and different interactions and reactions can occur depending on the concentration of oxygen in the headspace. Surprisingly, this was found even early after hermetically sealing the container and even at a level of oxygen which does not appear to be decisive regarding the oxidative degradation of noradrenaline, such as monitored by the formation of arterenone as the oxidative degradation product of noradrenaline. It was also surprisingly found that the presence of chelating agents appears to promote or accelerate the formation of subvisible particles specifically in a noradrenaline solution. As stated herein, within the present invention and its different aspects, the noradrenaline solution is free of chelating agents, as defined herein.

Regarding the drug product of the invention, as described herein, the preferred embodiments mentioned above for the process for producing a stable solution containing noradrenaline can be equally applied to the aspect of the invention concerning the process for reducing the number of subvisible particles in a solution containing noradrenaline. For example, the preferred oxygen concentration ranges in the headspace as well as the preferred volumes of the headspace (in relation to the noradrenaline solution) are as indicated above, in particular immediately after closing (sealing) the container. Also, the concentration of dissolved oxygen in the noradrenaline solution, immediately after sealing the container and upon equilibrium, and the concentration of noradrenaline in the solution are preferably as indicated above for the process for producing a stable solution containing noradrenalin.

The optimal reductions in the formation of subvisible particles were observed if the oxygen concentration in the headspace immediately after sealing the container is in the range from 0.15 to 1.0 percent v/v, in particular in the range of 0.2 to 0.95 percent v/v, more particular 0.3 to 0.8 percent v/v.

As mentioned above, according to a further preferred embodiment, the volume of the headspace is less than 11 %, preferably less than 10%, more preferably less than 9%, more preferably less than 8% of the volume of the solution containing noradrenaline, Optionally, the volume of the headspace is at least 1 % preferably at least 2.5 %, of the volume of the solution containing noradrenaline.

According to a further preferred embodiment of the invention, the drug product is in the form of a container which is an ampoule or vial, preferably a glass ampoule or vial.

As mentioned above, according to a further preferred embodiment, for the drug product of the invention, the concentration of noradrenaline in the noradrenaline containing solution is less than 0.1 mg/ml, preferably less than 0.06 mg/ml, more preferably less than 0.04 mg /ml, in particular less than 0.025 mg/ml. Particularly preferred, it is in the range between 0.001 to 0.04 mg/ml, to obtain the most significant effects on the low formation of subvisible particles. It is further preferred, that the drug product of the invention is free or substantially free of chelating agents, i.e. is completely free or has lower than 0.005% by weight, based on the total weight of the solution containing noradrenaline of chelating agents.

A further important aspect of the present invention is directed according to claims 12 and 13 to the use of a gas atmosphere comprising an oxygen concentration of less than 1.0 percent v/v, preferably in the range from 0.15 to 1.0 percent v/v, in particular in the range of 0.2 to 0.95 percent v/v, as a headspace in a container comprising a solution containing noradrenaline. As disclosed in detail above, the use of such a headspace with defined low oxygen content as specified herein has surprising advantages regarding the low formation of subvisible particles in a noradrenaline solution or a drug product containing a noradrenaline solution. Again, as disclosed in detail above, it is preferred that the oxygen concentration in the solution containing noradrenaline is less than 600 ppbw, in particular less than 340 ppbw, in particular less than 100 ppbw.

According to a further aspect of the present invention, the noradrenaline solution or drug product of the invention as described herein is used as a parenteral dosage form, in particular a ready-to-administer parenteral dosage form, preferably in the treatment of cardiac circulatory collapse, in states of shock associated with low peripheral resistances or to restore and/or keep physiological pressure levels.

Typically, the noradrenaline solution of the invention is a water-based solution and may be obtained by a process according to any one of the embodiments described herein. According to some embodiments of the present invention, the noradrenaline solution of the invention contains one or more an excipient(s), preferably the tonicity agent NaCl, in particular at a concentration in the range of 8.2 to 8.6 mg/ml, for example equal to 8.4 mg/ml.

The noradrenaline solution preferably includes a pharmaceutically acceptable tonicity agent and a pharmaceutically acceptable acid capable of adjusting the pH of the solution to 3.0 to 4.5. Suitable tonicity agents can be found in the United States Pharmacopoeia and include, for example, dextrose, glycerine, mannitol, potassium chloride and sodium chloride, with sodium chloride being most preferred. The tonicity agent is preferably present in concentrations adequate to render the solution isotonic, i.e. about 290 mOsm/kg, or substantially isotonic, i.e. from 250 to 350 mOsm/kg.

Preferably, the following definitions and use of terms apply:
As used in the specification and claims, the singular forms a, an, and the include plural references unless the context clearly dictates otherwise. For example, the term "a specification" refers to one or more specifications for use in the presently disclosed methods and systems. "A hydrocarbon" includes mixtures of two or more such hydrocarbons, and the like. The word "or" or like terms as used herein means any one member of a particular list and also includes any combination of members of that list.

As used in this specification and in the claims which follow, the word "comprise" and variations of the word, such as "comprising" and "comprises," means "including but not limited to," and is not intended to exclude, for example, other additives, components, integers or steps. When an element is described as comprising one or a plurality of components, steps or conditions, it will be understood that the element can also be described as "consisting of" or "consisting essentially of" the component, step or condition, or the plurality of components, steps or conditions.

When ranges are expressed herein by specifying alternative upper and lower limits of the range, it will be understood that the endpoints can be combined in any manner that is mathematically feasible. Thus, for example, a range of from 50 or 80 to 100 or 70 can alternatively be expressed as a series of ranges of from 50 to 100, from 50 to 70, and from 80 to 100. When a series of upper bounds and lower bounds are related using the phase "and" or "or", it will be understood that the upper bounds can be unlimited by the lower bounds or combined with the lower bounds, and vice versa. Thus, for example, a range of greater than 40% and/or less than 80% includes ranges of greater than 40%, less than 80%, and greater than 40% but less than 80%.

When an element of a process or thing is defined by reference to one or more examples, components, properties or characteristics, it will be understood that any one or combination of those components, properties or characteristics can also be used to define the subject matter at issue. This might occur, for example, when specific examples of an element are recited in a claim (as in a Markush grouping), or an element is defined by a plurality of characteristics. Thus, for example, if a claimed system comprises element A defined by elements A1, A2 and A3, in combination with element B defined by elements B1, B2 and B3, the invention will also be understood to cover a system defined by element A without element B, a system in which element A is defined by elements A1 and A2 in combination with element B defined by elements B2 and B3, and all other possible permutations.

When used herein, the term "about" will compensate for variability allowed for in the pharmaceutical industry and inherent in products in this industry, such as differences in product strength due to manufacturing variation and time-induced product degradation. The term allows for any variation which in the practice of good manufacturing practices would allow the product being evaluated to be considered therapeutically equivalent or bioequivalent in humans to the recited strength of a claimed product. In a preferred embodiment of the present invention, it encompasses ± 5 % of the respective indicated value.

The phrase "acceptable" as used in connection with compositions of the invention, refers to molecular entities and other ingredients of such compositions that are physiologically tolerable and do not typically produce untoward reactions when administered to a subject (e.g., a mammal such as a human).

When published test methodologies and diagnostic instruments are referred to herein, it will be understood that the test methodology or diagnostic instrument is performed based on the version in effect on October 1^{st}, 2020, unless otherwise stated to the contrary herein.

Also disclosed herein are the following embodiments i to xxiii (further aspects of the present invention), designated List A, noting that the scope of protection of the present invention is defined by the appended claims 1 to 17:
i. A process for producing a solution containing noradrenaline, in particular in the form of a drug product comprising the solution containing noradrenaline in a hermetically sealed container, comprising the following steps:
   a. dissolving noradrenaline and optionally one or more excipients in deoxygenated or degassed water, in order to produce a solution containing noradrenaline;
   b. distributing the noradrenaline solution in an inert gas current into a container,
   c. optionally sterilizing the solution containing noradrenaline,
   d. hermetically sealing the container,
   e. wherein the oxygen concentration in the solution containing noradrenaline is less than 300 ppbw immediately after sealing the container, and
   f. wherein the oxygen concentration in the headspace immediately after sealing the container is at most 1.0 percent v/v.
ii. A process according to embodiment i wherein the oxygen concentration in the solution containing noradrenaline is less than 100 ppbw, immediately after sealing the container.
iii. A process according to any of the preceding embodiments, wherein the solution containing noradrenaline is an injectable solution.
iv. A process according to any of the preceding embodiments, wherein the oxygen concentration in the solution containing noradrenaline in the hermetically sealed container upon equilibration at 25 °C is less than 600 ppbw, preferably less than 340 ppbw.
v. A process according to any of the preceding embodiments, wherein the oxygen concentration in the solution containing noradrenaline in the hermetically sealed container upon equilibration is determined according to the calculation method as described in Example 2.
vi. A process according to any of the preceding embodiments, wherein the oxygen concentration in the solution containing noradrenaline in the hermetically sealed container upon equilibration is determined by incubating the hermetically sealed container with the noradrenaline solution and the headspace at 25 °C for 7 days, preferably 14 days under ambient conditions.
vii. A process according to any of the preceding embodiments, wherein the oxygen concentration in the headspace immediately after sealing the container is less than 1.0 percent v/v, preferably 0.95 % v/v or less, even more preferably 0.9 % v/v or less, even more preferably 0.8 % v/v or less.
viii. A process according to any of the preceding embodiments, wherein the oxygen concentration in the headspace immediately after sealing the container is in the range from about 0.2 to about 1.0 percent v/v, preferably in the range of about 0.3 to about 0.95 percent v/v, in particular about 0.5 to about 0.95 percent v/v.
ix. A process according to any of the preceding embodiments, wherein the volume of the headspace is less than 11 %, preferably less than 10%, more preferably less than 9%, more preferably less than 8% of the volume of the solution containing noradrenaline.
x. A process according to any of the preceding embodiments, wherein the volume of the headspace is at least 1 % preferably at least 2.5 % of the volume of the solution containing noradrenaline.
xi. A process according to any of the preceding embodiments, wherein the container is an ampoule, vial, bottle, bag or pouch.
xii. A process according to any of the preceding embodiments, wherein the container is made of glass.
xiii. A process according to any of the preceding embodiments, wherein the container is made of a plastic material.
xiv. A process according to any of the preceding embodiments, wherein the concentration of noradrenaline in the noradrenaline solution is less than 1 mg/ml, preferably less than 0.1 mg, more preferably 0.06 mg/ml, even more preferably less than 0.04 mg /ml, in particular less than 0.025 mg/ml.
xv. A process according to any of the preceding embodiments, wherein the pH of the solution is in the range from 3.0 to 4.5, preferably 3.3 to 3.6, in particular 3.2 to 3.6.
xvi. A process according to any of the preceding embodiments, wherein the concentration of noradrenaline in the noradrenaline solution is in the range from 0.001 to 0.1 mg/ml, preferably 0.001 to 0.06 mg/ml, in particular 0.001 to 0.04 mg/ml.
xvii. A process according to any of the preceding embodiments, wherein sterilisation of the solution containing noradrenaline is performed by sterile filtration, heat treatment and/or irradiation, in particular sterile filtration and/or heat treatment.
xviii. A process according to any of the preceding embodiments, wherein a heat treatment for sterilisation is accomplished at temperatures above 100°C, for a time suitable for sterilization, in particular equal to or greater than 15 minutes, preferably a heat sterilization is performed for 15 minutes at 121 °C, in particular in an autoclave.
xix. A process according to any of the preceding embodiments, wherein a sterilization treatment of the solution containing noradrenaline is performed after hermetically sealing the container, by heat treatment.
xx. A process according to any of the preceding embodiments, wherein a sterilization treatment of the solution containing noradrenaline is performed by sterile filtration, before hermetically sealing the container, in particular before distributing the noradrenaline solution in an inert gas current into a container.
xxi. A process according to any of the preceding embodiments, wherein the solution containing noradrenaline is substantially free of antioxidizing agents (antioxidants), i.e. antioxidants are present in an amount less than 0.005% by weight, preferably less than 0.0005% by weight as determined by HPLC-MS; or wherein preferably the solution containing noradrenaline is free of antioxidizing agents (antioxidants), in particular free of sulfite(s), including bisulfite(s) and metabisulfite(s).
xxii. A process according to any of the preceding embodiments, wherein the solution containing noradrenaline is substantially free of chelating agents, i.e. chelating agents are present in an amount less than 0.005% by weight, preferably less than 0.0005% by weight as determined by HPLC-MS; or wherein preferably the solution containing noradrenaline is free of chelating agents, in particular free of EDTA (including edetic acid or any salt thereof such as disodium edetate), EGTA and/or DTPA.
xxiii. A process according to any of the preceding embodiments, wherein a batch of at least five drug products comprising the solution containing noradrenaline in a hermetically sealed container are produced, and wherein for each of the at least five drug products the oxygen concentration in the solution containing noradrenaline is less than 300 ppbw immediately after sealing the container, and the oxygen concentration in the headspace immediately after sealing the container is at most 1.0 percent v/v.

Further disclosed herein are the following embodiments i to xx directed to processes for reducing or limiting the number of subvisible particles in a solution containing noradrenaline, in particular in a hermetically sealed container (designated List B), noting that the scope of protection of the present invention is defined by the appended claims 1 to 17:
i. A process for reducing or limiting the number of subvisible particles in a solution containing noradrenaline, wherein the solution containing noradrenaline is contained in a container, together with a headspace, comprising the following steps:
   a. dissolving noradrenaline and optionally one or more excipients in deoxygenated or degassed water, in order to produce a solution containing noradrenaline;
   b. filling the solution containing noradrenaline into a container, the filled container comprising the solution containing noradrenaline and a headspace;
   c. controlling (or adjusting) the oxygen concentration in the headspace at less than 1.0 percent v/v.
ii. A process according to embodiment i, wherein the solution containing noradrenaline is filled into a container under inert gas flow, and the container is hermetically sealed after filling.
iii. A process according to any of the preceding embodiments, wherein the oxygen concentration in the solution containing noradrenaline is less than 300 ppbw, preferably less than 100 ppbw, immediately after sealing (closing) the container.
iv. A process according to any of the preceding embodiments, wherein the oxygen concentration in the solution containing noradrenaline in the hermetically sealed container upon equilibration at 25 °C is less than 600 ppbw, preferably less than 340 ppbw.
v. A process according to any of the preceding embodiments, wherein the oxygen concentration in the solution containing noradrenaline in the hermetically sealed container upon equilibration is determined according to the calculation method as described in Example 2.
vi. A process according to any of the preceding embodiments, wherein the oxygen concentration in the solution containing noradrenaline in the hermetically sealed container upon equilibration is determined by incubating the hermetically sealed container with the noradrenaline solution and the headspace at 25 °C for 7 days, preferably 14 days under ambient conditions.
vii. A process according to any of the preceding embodiments, wherein the oxygen concentration in the headspace is less than 1.0 percent v/v, preferably 0.95 % v/v or less, even more preferably 0.9 % v/v or less, even more preferably 0.8 % v/v or less.
viii. A process according to any of the preceding embodiments, wherein the oxygen concentration in the headspace is in the range from about 0.2 to about 1.0 % v/v, preferably in the range of about 0.3 to about 0.95 % v/v, in particular about 0.5 to about 0.95 % v/v.
ix. A process according to any of the preceding embodiments, wherein the volume of the headspace in the sealed (closed) container is less than 11 %, preferably less than 10%, more preferably less than 9%, more preferably less than 8%, of the volume of the solution containing noradrenaline.
x. A process according to any of the preceding embodiments, wherein the volume of the headspace in the sealed (closed) container is at least 1 % preferably at least 2.5 %, of the volume of the solution containing noradrenaline.
xi. A process according to any of the preceding embodiments, wherein the container is an ampoule, vial, bottle, bag or pouch.
xii. A process according to any of the preceding embodiments, wherein the container is made of glass.
xiii. A process according to any of the preceding embodiments, wherein the container is made of a plastic material.
xiv. A process according to any of the preceding embodiments, wherein the concentration of noradrenaline in the noradrenaline containing solution is less than 1 mg/ml, preferably less than 0.1 mg, more preferably 0.06 mg/ml, even more preferably less than 0.04 mg /ml, in particular less than 0.025 mg/ml.
xv. A process according to any of the preceding embodiments, wherein the pH of the solution containing noradrenaline is in the range from 3.0 to 4.5, preferably 3.3 to 3.6, in particular 3.2 to 3.6.
xvi. A process according to any of the preceding embodiments, wherein the concentration of noradrenaline in the noradrenaline containing solution is in the range from 0.001 to 0.1 mg/ml, preferably 0.001 to 0.06 mg/ml, in particular 0.001 to 0.04 mg/ml.
xvii. A process according to any of the preceding embodiments, wherein a sterilization treatment of the solution containing noradrenaline is performed before and/or after hermetically sealing the container.
xviii. A process according to any of the preceding embodiments, wherein the solution containing noradrenaline is substantially free of antioxidizing agents (antioxidants), i.e. antioxidants are present in an amount less than 0.005% by weight, preferably less than 0.0005% by weight as determined by HPLC-MS; or wherein preferably the solution containing noradrenaline is free of antioxidizing agents (antioxidants), in particular free of sulfite(s), including bisulfite(s) and metabisulfite(s).
xix. A process according to any of the preceding embodiments, wherein the solution containing noradrenaline is substantially free of chelating agents, i.e. chelating agents are present in an amount less than 0.005% by weight, preferably less than 0.0005% by weight as determined by HPLC-MS; or wherein preferably the solution containing noradrenaline is free of chelating agents, in particular free of EDTA (including edetic acid or any salt thereof such as disodium edetate), EGTA and/or DTPA.
xx. A process according to any of the preceding embodiments, wherein a batch of at least five drug products comprising the solution containing noradrenaline in a hermetically sealed container are produced, and wherein for each of the at least five drug products the oxygen concentration in the solution containing noradrenaline is less than 300 ppbw immediately after sealing the container, and the oxygen concentration in the headspace immediately after sealing the container is at most 1.0 percent v/v.

Further disclosed herein are embodiments i to xxiv directed to a drug product comprising or in the form of a container comprising a solution containing noradrenaline (designated List C), noting that the scope of protection of the present invention is defined by the appended claims 1 to 17. The drug product can also be considered as a pharmaceutical composition.
i. A drug product comprising a hermetically sealed container comprising a solution containing noradrenaline and a headspace;
   wherein oxygen concentration in the solution containing noradrenaline in the hermetically sealed container upon equilibration at 25 °C is less than 600 ppbw, in particular less than 340 ppbw.
ii. A drug product comprising a hermetically sealed container comprising a solution containing noradrenaline and a headspace, obtainable by a process wherein the oxygen concentration in the solution containing noradrenaline is less than 300 ppbw immediately after sealing the container; and wherein the oxygen concentration in the headspace immediately after sealing the container is less than 1.0 percent v/v,
   in particular obtainable by a process for producing a solution containing noradrenaline according to any one of the above embodiments i to xxiii (List A),
iii. A drug product according to embodiment i or ii wherein the oxygen concentration in the solution containing noradrenaline is less than 100 ppbw, immediately after sealing the container.
iv. A drug product according to any of the preceding embodiments, wherein the solution containing noradrenaline is an injectable solution.
v. A drug product according to any of the preceding embodiments, wherein the oxygen concentration in the solution containing noradrenaline in the hermetically sealed container upon equilibration is determined according to the calculation method as described in Example 2.
vi. A drug product according to any of the preceding embodiments, wherein the oxygen concentration in the solution containing noradrenaline in the hermetically sealed container upon equilibration is determined by incubating the hermetically sealed container with the noradrenaline solution and the headspace at 25 °C for 7 days, preferably 14 days under ambient conditions.
vii. A drug product according to any of the preceding embodiments, wherein the oxygen concentration in the headspace immediately after sealing the container is less than 1.0 percent v/v, preferably 0.95 % v/v or less, even more preferably 0.9 % v/v or less, even more preferably 0.8 % v/v or less.
viii. A drug product according to any of the preceding embodiments, wherein the oxygen concentration in the headspace immediately after sealing the container is in the range from about 0.2 to about 1.0 percent v/v, preferably in the range of about 0.3 to about 0.95 percent v/v, in particular about 0.5 to about 0.95 percent v/v.
ix. A drug product according to any of the preceding embodiments, wherein the volume of the headspace is less than 11 %, preferably less than 10%, more preferably less than 9%, more preferably less than 8% of the volume of the solution containing noradrenaline.
x. A drug product according to any of the preceding embodiments, wherein the volume of the headspace is at least 1 % preferably at least 2.5 % of the volume of the solution containing noradrenaline.
xi. A drug product according to any of the preceding embodiments, wherein the container is an ampoule, vial, bottle, bag or pouch.
xii. A drug product according to any of the preceding embodiments, wherein the container is made of glass.
xiii. A drug product according to any of the preceding embodiments, wherein the container is made of a plastic material.
xiv. A drug product according to any of the preceding embodiments, wherein the concentration of noradrenaline in the noradrenaline containing solution is less than 1 mg/ml, preferably less than 0.1 mg, more preferably 0.06 mg/ml, even more preferably less than 0.04 mg/ml, in particular less than 0.025 mg/ml.
xv. A drug product according to any of the preceding embodiments, wherein the pH of the solution is in the range from 3.0 to 4.5, preferably 3.3 to 3.6, in particular 3.2 to 3.6.
xvi. A drug product according to any of the preceding embodiments, wherein the concentration of noradrenaline in the noradrenaline containing solution is in the range from 0.001 to 0.1 mg/ml, preferably 0.001 to 0.06 mg/ml, in particular 0.001 to 0.04 mg/ml.
xvii. A sterile drug product according to any of the preceding embodiments, wherein sterilization of the solution containing noradrenaline is performed by sterile filtration, heat treatment and/or irradiation, in particular sterile filtration and/or heat treatment.
xviii. A sterile drug product according to any of the preceding embodiments, wherein a heat treatment for sterilization is accomplished at temperatures above 100°C, for a time suitable for sterilization, in particular equal to or greater than 15 minutes, preferably a heat sterilization is performed for 15 minutes at 121 °C, in particular in an autoclave.
xix. A sterile drug product according to any of the preceding embodiments, wherein a sterilization treatment of the solution containing noradrenaline is performed after hermetically sealing the container, by heat treatment.
xx. A sterile drug product according to any of the preceding embodiments, wherein a sterilization treatment of the solution containing noradrenaline is performed by sterile filtration, before hermetically sealing the container, in particular before distributing the noradrenaline solution in an inert gas current into a container.
xxi. A drug product according to any of the preceding embodiments, wherein the solution containing noradrenaline is substantially free of antioxidizing agents (antioxidants), i.e. antioxidants are present in an amount less than 0.005% by weight, preferably less than 0.0005% by weight as determined by HPLC-MS; or wherein preferably the solution containing noradrenaline is free of antioxidizing agents (antioxidants), in particular free of sulfite(s), including bisulfite(s) and metabisulfite(s).
xxii. A drug product according to any of the preceding embodiments, wherein the solution containing noradrenaline is substantially free of chelating agents, i.e. chelating agents are present in an amount less than 0.005% by weight, preferably less than 0.0005% by weight as determined by HPLC-MS; or wherein preferably the solution containing noradrenaline is free of chelating agents, in particular free of EDTA (including edetic acid or any salt thereof such as disodium edetate), EGTA and/or DTPA.
xxiii. A drug product according to any of the preceding embodiments, obtainable by a process according to any one of embodiments i to xxiii of List A above.
xxiv. A batch of at least five drug products according to any of the preceding claims, wherein for each of the at least five drug products the oxygen concentration in the solution containing noradrenaline is less than 300 ppbw immediately after sealing the container, and the oxygen concentration in the headspace immediately after sealing the container is at most 1.0 percent v/v.

Further disclosed is the use of a drug product according to any of the afore-mentioned embodiments i toxxiv (List C) for use as a medicament as a parenteral dosage form, in particular a ready-to-administer parenteral dosage form, preferably in the treatment of cardiac circulatory collapse, in states of shock associated with low peripheral resistances or to restore and/or keep physiological pressure levels.

A further disclosure concerns a drug product according to any of the afore-mentioned embodiments i to xxiv (List C) as a parenteral dosage form, in particular a ready-to-administer parenteral dosage form, noting that the scope of protection of the present invention is defined by the appended claims 1 to 17. A further disclosure concerns a drug product according to any of the afore-mentioned embodiments i to xxiv (List C) for use in the treatment of cardiac circulatory collapse, in states of shock associated with low peripheral resistances or to restore and/or keep physiological pressure levels, noting that the scope of protection of the present invention is defined by the appended claims 1 to 17.

Further disclosed herein are embodiments i to xxiii directed to a use of a gas atmosphere as a headspace in a container comprising a solution containing noradrenaline (designated List D), noting that the scope of protection of the present invention is defined by the appended claims 1 to 17:
i. Use of a gas atmosphere comprising an oxygen concentration of 1.0 percent v/v or less, as a headspace in a container comprising a solution containing noradrenaline, in particular for reducing or limiting the formation of subvisible particles.
ii. A use according to embodiment i, wherein the container is hermetically sealed.
iii. A use according to embodiment i or ii, wherein the oxygen concentration in the solution containing noradrenaline is less than 300 ppbw, in particular less than 100 ppbw, in particular immediately after hermetically sealing the container.
iv. A use according to any of the preceding embodiments, wherein the solution containing noradrenaline is an injectable solution, which can be administered parentally to a patient.
v. A use according to any of the preceding embodiments, wherein the oxygen concentration in the solution containing noradrenaline in the hermetically sealed container upon equilibration at 25 °C is less than 600 ppbw, preferably less than 340 ppbw.
vi. A use according to any of the preceding embodiments, wherein the oxygen concentration in the solution containing noradrenaline in the hermetically sealed container upon equilibration is determined according to the calculation method as described in Example 2.
vii. A use according to any of the preceding embodiments, wherein the oxygen concentration in the solution containing noradrenaline in the hermetically sealed container upon equilibration is determined by incubating the hermetically sealed container with the noradrenaline solution and the headspace at 25 °C for 7 days, preferably 14 days under ambient conditions.
viii. A use according to any of the preceding embodiments, wherein the oxygen concentration in the headspace, is less than 1.0 percent v/v, preferably 0.95 % v/v or less, even more preferably 0.9 % v/v or less, even more preferably 0.8 % v/v or less, in particular immediately after sealing the container.
ix. A use according to any of the preceding embodiments, wherein the oxygen concentration in the headspace, is in the range from about 0.2 to about 1.0 percent v/v, preferably in the range of about 0.3 to about 0.95 percent v/v, in particular about 0.5 to about 0.95 percent v/v, in particular immediately after sealing the container.
x. A use according to any of the preceding embodiments, wherein the volume of the headspace is less than 11 %, preferably less than 10%, more preferably less than 9%, more preferably less than 8% of the volume of the solution containing noradrenaline.
xi. A use according to any of the preceding embodiments, wherein the volume of the headspace is at least 1 % preferably at least 2.5 % of the volume of the solution containing noradrenaline.
xii. A use according to any of the preceding embodiments, wherein the container is an ampoule, vial, bottle, bag or pouch.
xiii. A use according to any of the preceding embodiments, wherein the container is made of glass and/or a plastic material.
xiv. A use according to any of the preceding embodiments, wherein the concentration of noradrenaline in the noradrenaline containing solution is less than 1 mg/ml, preferably less than 0.1 mg, more preferably 0.06 mg/ml, even more preferably less than 0.04 mg /ml, in particular less than 0.025 mg/ml.
xv. A use according to any of the preceding embodiments, wherein the pH of the solution is in the range from 3.0 to 4.5, preferably 3.3 to 3.6, in particular 3.2 to 3.6.
xvi. A use according to any of the preceding embodiments, wherein the concentration of noradrenaline in the noradrenaline containing solution is in the range from 0.001 to 0.1 mg/ml, preferably 0.001 to 0.06 mg/ml, in particular 0.001 to 0.04 mg/ml.
xvii. A process according to any of the preceding embodiments, wherein sterilisation of the solution containing noradrenaline is performed by sterile filtration, heat treatment and/or irradiation, in particular sterile filtration and/or heat treatment.
xviii. A use according to any of the preceding embodiments, wherein a heat treatment for sterilization is accomplished at temperatures above 100°C, for a time suitable for sterilization, in particular equal to or greater than 15 minutes, preferably a heat sterilization is performed for 15 minutes at 121 °C, in particular in an autoclave.
xix. A use according to any of the preceding embodiments, wherein a sterilization treatment of the solution containing noradrenaline is performed after hermetically sealing the container, by heat treatment.
xx. A use according to any of the preceding embodiments, wherein a sterilization treatment of the solution containing noradrenaline is performed by sterile filtration, before hermetically sealing the container, in particular before distributing the noradrenaline solution in an inert gas current into a container.
xxi. A use according to any of the preceding embodiments, wherein the solution containing noradrenaline is substantially free of antioxidizing agents (antioxidants), i.e. antioxidants are present in an amount less than 0.005% by weight, preferably less than 0.0005% by weight as determined by HPLC-MS; or wherein preferably the solution containing noradrenaline is free of antioxidizing agents (antioxidants), in particular free of sulfite(s), including bisulfite(s) and metabisulfite(s).
xxii. A use according to any of the preceding embodiments, wherein the solution containing noradrenaline is substantially free of chelating agents, i.e. chelating agents are present in an amount less than 0.005% by weight, preferably less than 0.0005% by weight as determined by HPLC-MS; or wherein preferably the solution containing noradrenaline is free of chelating agents, in particular free of EDTA (including edetic acid or any salt thereof such as disodium edetate), EGTA and/or DTPA.
xxiii. A use according to any of the preceding embodiments, in a batch of at least five drug product comprising the solution containing noradrenaline in a hermetically sealed container, wherein for each of the at least five drug products the oxygen concentration in the solution containing noradrenaline is less than 300 ppbw immediately after sealing the container, and the oxygen concentration in the headspace immediately after sealing the container is at most 1.0 percent v/v.

The invention will now be illustrated, without limitation, by the following Examples:

### EXAMPLES

### Example 1: Effect of Oxygen Concentrations on Oxidation By-Products and Subvisible Particle Formation

A study was undertaken to evaluate the effect of oxygen content in the headspace of 50 ml noradrenaline bitartrate vials on product stability, analyzing for noradrenaline bitartrate (NA) concentration (assay %) oxygen (% v/v), subvisible particle counts ≥ 10 µm, subvisible particle counts ≥ 25 µm, arterenone concentration (mg/ml), and D-noradrenaline (D-NA) concentration at different time points. As mentioned above, particle counts were measured using the light obscuration particle count test prescribed by USP chapter 788 (official as of 1 May 2013). All testing was undertaken according to stability methods prescribed by the International Conference on Harmonization, ICH Q1A (R2) (effective 1 August 2003).

Batches of 50 ml glass vials were manufactured at noradrenaline concentrations ranging from 0.06 mg/ml to 0.2 mg/ml. Sixteen (16) separate production batches were evaluated for this Example; between 16 and 160 samples were withdrawn from each production batch, and evaluated for oxygen content in the headspace shortly after filling. The oxygen contents of the samples were then summed and averaged to give an average oxygen content for each batch. Vials were filled in an inert nitrogen atmosphere to an average solution volume of 53.5 ml, with an average headspace volume of 3.5 ml (i.e. 6.25%). The solution itself was purged with nitrogen prior to ingredient dissolution and filling into the vials, and constantly kept under strict exclusion of oxygen/air such that oxygen content in the final solution was consistently maintained below 100 ppb, in fact below 50 ppbw, including the use of a coaxial needle for filling the vials with the noradrenaline solution and a stream of nitrogen at the same time.

Product was manufactured aseptically, without a high temperature terminal sterilization, and without protection from light. 50 ml vials used for this Example were Type I clear colorless glass vials closed with a bromo-butyl stopper and an aluminum flip-off cap. The formulations consisted of noradrenaline bitartrate, sodium chloride, hydrochloric acid 1 N (for pH-adjustment), and water for injection. The final pH of the solution was 3.0-4.5. The final osmolarity was 250 - 350 mOsm/kg. The concentration of dissolved oxygen in the noradrenaline solution was below 100 ppbw immediately after sealing the vials.

Results for room temperature stability testing (25 °C, 60% RH) and accelerated stability testing (40 °C, 75% RH) at the 24-month and 6-month time points are presented below in Tables 1a and 1b. The results are divided between high O₂ headspace batches (> 1% v/v) and low O₂ headspace batches (< 1% v/v). The oxygen concentrations were determined as disclosed herein, in particular Frequency Modulation Spectroscopy (FMS), or using a 3600 Series Analyzer for Oxygen (Orbisphere, CH) according to the manufacturer's instructions.

The headspace contained, apart from oxygen (as indicated below) and nitrogen only argon (<0.1 %v/v). Other gases (traces of components from air) were below 0.005 % v/v.

The equilibrium oxygen concentration in solution, when calculated according to the method of Example 2 below, was consistently less than 340 ppb for the upper section of Tables 1a and b (low O₂ headspace batches having an oxygen content of the headspace less than 1% v/v), and consistently above 340 ppb for the lower section of Tables 1 a and b (high O₂ headspace batches having an oxygen content of the headspace more than 1 % v/v).

**Table 1a (25 °C, 60% RH, 24 months)**

| NA Conc. (mg/ml) | Headspace O₂ (% v/v; T₀)** | Particles* ≥ 10 µm | Particles* ≥ 25µm | Arterenone |
|---|---|---|---|---|
| 0.06 | 0.41 | 1313 | 33 | 0.1 |
| 0.1 | 0.49 | 613 | 2 | 0.06 |
| 0.12 | 0.58 | 1055 | 10 | 0.06 |
| 0.2 | 0.47 | 1880 | 38 | 0.05 |
| 0.2 | 0.39 | 2472 | 32 | |
| 0.1 | 1.77 | 7228 | 118 | 0.06 |
| 0.1 | 1.26 | 7302 | 216 | 0.06 |
| 0.2 | 1.21 | 6626 | 110 | 0.06 |
| 0.2 | 1.50 | 13845 | 455 | 0.06 |
| 0.2 | 1.59 | 8478 | 356 | 0.07 |

| | | | | |
|---|---|---|---|---|
| ** T₀ = immediately after sealing the container (vial); *particle counts are particles per 50 ml vial | | | | |

**Table 1b (40 °C, 75% RH, 6 months)**

| NA Conc. (mg/ml) | Headspace O₂ (% v/v; T₀)** | Particles* ≥ 10 µm | Particles* ≥ 25µm | Arterenone | O₂ in solution at equilibrium (25°C)*** |
|---|---|---|---|---|---|
| 0.06 | 0.41 | 1145 | 13 | 0.11 | 142 |
| 0.1 | 0.49 | 386 | 6 | 0.09 | 166 |
| 0.1 | 0.59 | 532 | 5 | 0.12 | 197 |
| 0.1 | 0.72 | 992 | 48 | 0.07 | 237 |
| 0.12 | 0.52 | 693 | 19 | 0.07 | 176 |
| 0.12 | 0.58 | 750 | 24 | 0.09 | 194 |
| 0.2 | 0.64 | 832 | 22 | 0.04 | 213 |
| 0.2 | 0.39 | 1417 | 19 | 0.06 | 136 |
| 0.1 | 1.42 | 5090 | 124 | 0.07 | 452 |
| 0.1 | 1.26 | 5700 | 297 | 0.04 | 403 |
| 0.2 | 1.21 | 5157 | 207 | 0.03 | 388 |
| 0.2 | 1.50 | 4567 | 237 | 0.07 | 477 |
| 0.2 | 1.59 | 7887 | 397 | 0.1 | 504 |

| | | | | | |
|---|---|---|---|---|---|
| *particle counts are particles per 50 ml vial; ** T₀ = immediately after sealing the container (vial); *** measured according to the method in Example 2, immediately after sealing the container (vial) | | | | | |

As evident from Tables 1a and 1b, based on the arterenone data, oxidation was not affected by whether the batch had a high oxygen concentration in the headspace or a low oxygen concentration in the headspace.

However, subvisible particle formation was greatly affected by the concentration of oxygen in the headspace, and improved at lower oxygen concentrations. This discovery was surprising, and has tremendous practical significance for the production of long-term stable, injectable noradrenaline solutions, even at low concentration.

It was also found that in particular the early formation of subvisible particles was reduced by using a headspace with an oxygen content of less than 1% v/v immediately after sealing the vials. As can be seen from Table 1c below, the subvisible particles did not increase in the first three months of storage, if an oxygen content of less than 1% v/v oxygen in the headspace was used. In fact, the number of subvisible particles significantly decreased when comparing the counts immediately after sealing the container, and after 3 months storage, if using a headspace with an oxygen content of less than 1% v/v immediately after sealing the vials. Some of the smallest subvisible particles even appeared to re-dissolve.

**Table 1c (25 °C, 60% RH storage)**

| NA Conc. (mg/ml) | Headspace O₂ (% v/v; T₀) | Particles ≥ 10 µm at T₃ₘₒ as % of particles ≥ 10 µm at T₀* |
|---|---|---|
| 0.06 | 0.41 | 5 % |
| 0.1 | 0.49 | 39 % |
| 0.12 | 0.58 | 2 % |
| 0.2 | 0.47 | 18 % |
| 0.2 | 0.39 | 10 % |
| 0.1 | 1.77 | 648 % |
| 0.1 | 1.26 | 475 % |
| 0.2 | 1.21 | 1033 % |
| 0.2 | 1.50 | 1681 % |
| 0.2 | 1.59 | 1060 % |

| | | |
|---|---|---|
| *particle counts are particles per 50 ml vial; T₀ = immediately after sealing the container (vial); T₃ₘₒ = at 3 months after sealing the container (vial); | | |

### Example 2: Calculation of Oxygen Concentrations in Headspace and in the Solution Volume at Equilibrium

The following procedure can be used to determine the concentrations of oxygen in solution and in the headspace in a sealed container after the oxygen between these two phases has been allowed to equilibrate. This method can be used regardless of the container selected or the volume inside the container, as long as the oxygen in the headspace is initially greater than the oxygen in the headspace at equilibrium. Calculation is in fact routine practice of the skilled person and applies two well-known laws, the Henry's law (see e.g. https://goldbook.iupac.org/terms/view/H02783), and the chemical equilibrium law.

According to this example assumes a liquid volume of 50 mL and a gaseous headspace volume of 6 mL, a dimensionless Henry's Law constant (K_{H}) of 3.2×10⁻², an initial amount of oxygen in water of 5 µg, and an initial amount of oxygen in the gaseous headspace of 85 µg. Dimensionless Henry's constants K for oxygen, nitrogen and argon in water are 3.2×10⁻², 1.5×10⁻², and 3.4×10⁻², respectively. Equilibrium equation can be depicted as follows (wherein "Oxygen (g)" is oxygen in the gas phase (headspace), and "Oxygen (aq)" is oxygen in the solution):

Oxygen (g) ↔ Oxygen (aq)

Assuming no or negligible chemical or biological loss, equilibrium laws indicate an x increase for the right-hand side of the equation compensating an identical decrease for the left-hand part, all governed by equilibrium constant: K = [[mol(aq) + x]/V(aq) ] / [[mol(g) - x]/V(g)]

Solving per x, and adding it to the initial contribution in water, one finds a final dissolved concentration of 381 micrograms/L.

## Claims

1. A process for producing a stable, injectable solution containing noradrenaline, wherein the solution of noradrenaline is free of antioxidants, comprising the following steps:
a. dissolving noradrenaline and optionally one or more excipients in deoxygenated or degassed water, in order to produce a solution containing noradrenaline;
b. distributing the noradrenaline solution in an inert gas current into a container,
c. sterilizing the solution containing noradrenaline, before and/or after step b),
d. hermetically sealing the container,
e. wherein the oxygen concentration in the solution containing noradrenaline is less than 300 ppbw immediately after sealing the container; and
f. wherein the oxygen concentration in the headspace immediately after sealing the container is at most 1.0 percent v/v.

2. A process in accordance with claim 1 wherein oxygen concentration in the solution containing noradrenaline in the hermetically sealed container upon equilibration at 25 °C is less than 600 ppbw, preferably less than 340 ppbw.

3. A process according to claim 1 or 2, wherein the oxygen concentration in the headspace immediately after sealing the container is in the range from 0.15 to 1.0 percent v/v, preferably in the range from 0.2 to 1.0 percent v/v, in particular in the range of 0.3 to 0.95 percent v/v.

4. A process according to any one of claims 1 to 3, wherein the volume of the headspace is less than 11 %, preferably less than 10%, more preferably less than 9%, more preferably less than 8% of the volume of the solution containing noradrenaline, and optionally wherein the volume of the headspace is at least 1 % preferably at least 2.5 % of the volume of the solution containing noradrenaline.

5. A process according to any one of claims 1 to 4, wherein the concentration of noradrenaline in the noradrenaline containing solution is less than 0.1 mg/ml, preferably less than 0.06 mg/ml, more preferably less than 0.04 mg /ml, in particular less than 0.025 mg/ml, further in particular in the range between 0.001 to 0.04 mg/ml.

6. A process for reducing the number of subvisible particles in a solution containing noradrenaline, wherein the solution of noradrenaline is free of antioxidants, and wherein the solution containing noradrenaline is contained in a container, together with a headspace, comprising the following steps:
a. dissolving noradrenaline and optionally one or more excipients in deoxygenated or degassed water, in order to produce a solution containing noradrenaline;
b. filling the solution containing noradrenaline into a container, the filled container comprising the solution containing noradrenaline and a headspace;
c. controlling the oxygen concentration in the headspace at equal to or less than 1.0 percent v/v, preferably in the range from 0.15 to 1.0 percent v/v, in particular in the range of 0.2 to 0.95 percent v/v.

7. A drug product comprising:
a hermetically sealed container comprising a solution containing noradrenaline and a headspace,
obtainable by a process wherein the oxygen concentration in the solution containing noradrenaline immediately after sealing the container is less than 300 ppbw, in particular less than 100 ppbw, and wherein the oxygen concentration in the headspace immediately after sealing the container is equal to or less than 1.0 percent v/v,
in particular obtainable by a process according to any one of claims 1 to 6,
wherein the solution of noradrenaline is free of antioxidants.

8. The drug product according to claim 7, wherein oxygen concentration in the solution containing noradrenaline in the hermetically sealed container upon equilibration at 25 °C is less than 600 ppbw, in particular less than 340 ppbw.

9. The drug product according to claim 7 or 8, wherein the oxygen concentration in the headspace immediately after sealing the container is in the range from 0.15 to 1.0 percent v/v, preferably in the range from 0.2 to 1.0 percent v/v, in particular in the range of 0.3 to 0.95 percent v/v.

10. The drug product according to any one of claims 7 to 9, wherein the volume of the headspace is less than 11 %, preferably less than 10%, more preferably less than 9%, more preferably less than 8% of the volume of the solution containing noradrenaline, and optionally wherein the volume of the headspace is at least 1 % preferably at least 2.5 %, of the volume of the solution containing noradrenaline.

11. The drug product according to any one of claims 7 to 10, wherein the concentration of noradrenaline in the noradrenaline containing solution is less than 0.1 mg/ml, preferably less than 0.06 mg/ml, more preferably less than 0.04 mg /ml, in particular less than 0.025 mg/ml, further in particular in the range between 0.001 to 0.04 mg/ml.

12. Use of a gas atmosphere comprising an oxygen concentration of equal to or less than 1.0 percent v/v, preferably in the range from 0.15 to 1.0 percent v/v, in particular in the range of 0.2 to 0.95 percent v/v, as a headspace in a container comprising a solution containing noradrenaline, wherein the solution of noradrenaline is free of antioxidants, for reducing the formation of subvisible particles in the solution.

13. Use according to the preceding claim, wherein the oxygen concentration in the solution containing noradrenaline is less than 600 ppbw, in particular less than 340 ppbw, in particular less than 100 ppbw.

14. Drug product according to any one of claims 7 to 11, or produced according to a method according to any one of claims 1 to 6 for use as a medicament in a parenteral dosage form, in particular a ready-to-administer parenteral dosage form, preferably in the treatment of cardiac circulatory collapse, in states of shock associated with low peripheral resistances or to restore and/or keep physiological pressure levels.

15. A process according to any one of claims 1 to 6 wherein the solution of noradrenaline is free of chelating agents.

16. A drug product according to any one of claims 7 to 11 wherein the solution of noradrenaline is free of chelating agents.

17. Use according to claim 12 or 13 wherein the solution of noradrenaline is free of chelating agents.

## Patentansprüche

1. Verfahren zum Herstellen einer stabilen, injizierbaren Lösung enthaltend Noradrenalin, wobei die Lösung von Noradrenalin frei von Antioxidantien ist, umfassend die folgenden Schritte:
a. Lösen von Noradrenalin und gegebenenfalls einem oder mehr Exzipienten in deoxygeniertem oder entgastem Wasser, um eine Noradrenalin enthaltende Lösung herzustellen;
b. Verteilen der Noradrenalinlösung in einem Inertgasstrom in einen Behälter,
c. Sterilisieren der Noradrenalin enthaltenden Lösung, vor und/oder nach Schritt b),
d. hermetisch Versiegeln des Behälters,
e. wobei die Sauerstoffkonzentration in der Noradrenalin enthaltenden Lösung unmittelbar nach dem Versiegeln des Behälters weniger als 300 ppbw beträgt, und
f. wobei die Sauerstoffkonzentration im Kopfraum unmittelbar nach dem Versiegeln des Behälters höchstens 1,0 Prozent v/v beträgt.

2. Verfahren gemäß Anspruch 1, wobei die Sauerstoffkonzentration in der Noradrenalin enthaltenden Lösung in dem hermetisch versiegelten Behälter nach Gleichgewichtseinstellung bei 25 °C weniger als 600 ppbw, bevorzugt weniger als 340 ppbw beträgt.

3. Verfahren gemäß Anspruch 1 oder 2, wobei die Sauerstoffkonzentration in dem Kopfraum unmittelbar nach dem Versiegeln des Behälters im Bereich von 0,15 bis 1,0 Prozent v/v, bevorzugt im Bereich von 0,2 bis 1,0 Prozent v/v, insbesondere im Bereich von 0,3 bis 0,95 Prozent v/v liegt.

4. Verfahren gemäß einem der Ansprüche 1 bis 3, wobei das Volumen des Kopfraums weniger als 11 %, bevorzugt weniger als 10 %, stärker bevorzugt weniger als 9 %, stärker bevorzugt weniger als 8 % des Volumens der Noradrenalin enthaltenden Lösung beträgt, und wobei gegebenenfalls das Volumen des Kopfraums mindestens 1 %, bevorzugt mindestens 2,5 % des Volumens der Noradrenalin enthaltenden Lösung beträgt.

5. Verfahren gemäß einem der Ansprüche 1 bis 4, wobei die Konzentration an Noradrenalin in der Noradrenalin enthaltenden Lösung weniger als 0,1 mg/ml, bevorzugt weniger als 0,06 mg/ml, stärker bevorzugt weniger als 0,04 mg/ml, insbesondere weniger als 0,025 mg/ml beträgt, ferner insbesondere im Bereich zwischen 0,001 bis 0,04 mg/ml liegt.

6. Verfahren zum Verringern der Anzahl von nicht-sichtbaren Partikeln in einer Noradrenalin enthaltenden Lösung, wobei die Lösung von Noradrenalin frei von Antioxidantien ist, und wobei die Noradrenalin enthaltende Lösung in einem Behälter, zusammen mit einem Kopfraum, enthalten ist, umfassend die folgenden Schritte:
a. Lösen von Noradrenalin und gegebenenfalls einem oder mehr Exzipienten in deoxygeniertem oder entgastem Wasser, um eine Noradrenalin enthaltende Lösung herzustellen;
b. Füllen der Noradrenalin enthaltenden Lösung in einen Behälter, wobei der gefüllte Behälter die Noradrenalin enthaltende Lösung und einen Kopfraum umfasst;
c. Steuern der Sauerstoffkonzentration in dem Kopfraum bei gleich oder niedriger als 1,0 Prozent v/v, bevorzugt im Bereich von 0,15 bis 1,0 Prozent v/v, insbesondere im Bereich von 0,2 bis 0,95 Prozent v/v.

7. Arzneistoffprodukt umfassend:
einen hermetisch versiegelten Behälter umfassend eine Noradrenalin enthaltende Lösung und einen Kopfraum,
erhältlich durch ein Verfahren, wobei die Sauerstoffkonzentration in der Noradrenalin enthaltenden Lösung unmittelbar nach dem Versiegeln des Behälters weniger als 300 ppbw, insbesondere weniger als 100 ppbw beträgt, und wobei die Sauerstoffkonzentration in dem Kopfraum unmittelbar nach dem Versiegeln des Behälters gleich oder niedriger als 1,0 Prozent v/v ist,
insbesondere erhältlich durch ein Verfahren gemäß einem der Ansprüche 1 bis 6,
wobei die Lösung von Noradrenalin frei von Antioxidantien ist.

8. Arzneistoffprodukt gemäß Anspruch 7, wobei die Sauerstoffkonzentration in der Noradrenalin enthaltenden Lösung in dem hermetisch versiegelten Behälter nach Gleichgewichtseinstellung bei 25 °C weniger als 600 ppbw, insbesondere weniger als 340 ppbw beträgt.

9. Arzneistoffprodukt gemäß Anspruch 7 oder 8, wobei die Sauerstoffkonzentration in dem Kopfraum unmittelbar nach dem Versiegeln des Behälters im Bereich von 0,15 bis 1,0 Prozent v/v, bevorzugt im Bereich von 0,2 bis 1,0 Prozent v/v, insbesondere im Bereich von 0,3 bis 0,95 Prozent v/v liegt.

10. Arzneistoffprodukt gemäß einem der Ansprüche 7 bis 9, wobei das Volumen des Kopfraums weniger als 11 %, bevorzugt weniger als 10 %, stärker bevorzugt weniger als 9 %, stärker bevorzugt weniger als 8 % des Volumens der Noradrenalin enthaltenden Lösung beträgt, und wobei gegebenenfalls das Volumen des Kopfraums mindestens 1 %, bevorzugt mindestens 2,5 % des Volumens der Noradrenalin enthaltenden Lösung beträgt.

11. Arzneistoffprodukt gemäß einem der Ansprüche 7 bis 10, wobei die Konzentration an Noradrenalin in der Noradrenalin enthaltenden Lösung weniger als 0,1 mg/ml, bevorzugt weniger als 0,06 mg/ml, stärker bevorzugt weniger als 0,04 mg/ml, insbesondere weniger als 0,025 mg/ml beträgt, ferner insbesondere im Bereich zwischen 0,001 bis 0,04 mg/ml liegt.

12. Verwendung einer Gasatmosphäre, umfassend eine Sauerstoffkonzentration von gleich oder niedriger als 1,0 Prozent v/v, bevorzugt im Bereich von 0,15 bis 1,0 Prozent v/v, insbesondere im Bereich von 0,2 bis 0,95 Prozent v/v, als ein Kopfraum in einem Behälter umfassend eine Noradrenalin enthaltende Lösung, wobei die Lösung von Noradrenalin frei von Antioxidantien ist, zum Verringern der Bildung von nicht-sichtbaren Partikeln in der Lösung.

13. Verwendung gemäß dem vorhergehenden Anspruch, wobei die Sauerstoffkonzentration in der Noradrenalin enthaltenden Lösung weniger als 600 ppbw, insbesondere weniger als 340 ppbw, insbesondere weniger als 100 ppbw beträgt.

14. Arzneistoffprodukt gemäß einem der Ansprüche 7 bis 11, oder hergestellt gemäß einem Verfahren gemäß einem der Ansprüche 1 bis 6 zur Verwendung als ein Medikament in einer parenteralen Dosierungsform, insbesondere einer verabreichungsbereiten parenteralen Dosierungsform, bevorzugt in der Behandlung von Herzkreislaufkollaps, bei Schockzuständen in Zusammenhang mit niedrigen peripheren Widerständen oder zum Wiederherstellen und/oder Aufrechterhalten von physiologischen Drucklevels.

15. Verfahren gemäß einem der Ansprüche 1 bis 6, wobei die Lösung von Noradrenalin frei von chelatbildenden Mitteln ist.

16. Arzneistoffprodukt gemäß einem der Ansprüche 7 bis 11, wobei die Lösung von Noradrenalin frei von chelatbildenden Mitteln ist.

17. Verwendung gemäß Anspruch 12 oder 13, wobei die Lösung von Noradrenalin frei von chelatbildenden Mitteln ist.

## Revendications

1. Procédé de production d'une solution injectable stable contenant de la noradrénaline, dans lequel la solution de noradrénaline est exempte d'antioxydants, comprenant les étapes suivantes :
a. la dissolution de la noradrénaline et éventuellement d'un ou de plusieurs excipients dans de l'eau désoxygénée ou dégazée, afin de produire une solution contenant de la noradrénaline ;
b. la distribution de la solution de noradrénaline dans un courant de gaz inerte dans un récipient,
c. la stérilisation de la solution contenant de la noradrénaline, avant et/ou après l'étape b),
d. le scellement hermétique du récipient,
e. dans lequel la concentration d'oxygène dans la solution contenant de la noradrénaline est inférieure à 300 ppb en poids immédiatement après le scellement du récipient ; et
f. dans lequel la concentration d'oxygène dans l'espace libre immédiatement après le scellement du récipient est d'au plus 1,0 pour cent v/v.

2. Procédé selon la revendication 1, dans lequel la concentration d'oxygène dans la solution contenant de la noradrénaline dans le récipient hermétiquement scellé après équilibrage à 25°C est inférieure à 600 ppb en poids, de préférence inférieure à 340 ppb en poids.

3. Procédé selon la revendication 1 ou 2, dans lequel la concentration d'oxygène dans l'espace libre immédiatement après le scellement du récipient se trouve dans la plage allant de 0,15 à 1,0 pour cent v/v, de préférence dans la plage allant de 0,2 à 1,0 pour cent v/v, en particulier dans la plage allant de 0,3 à 0,95 pour cent v/v.

4. Procédé selon l'une quelconque des revendications 1 à 3, dans lequel le volume de l'espace libre est inférieur à 11%, de préférence inférieur à 10%, plus préférablement inférieur à 9%, plus préférablement inférieur à 8% du volume de la solution contenant de la noradrénaline, et éventuellement dans lequel le volume de l'espace libre est d'au moins 1%, de préférence d'au moins 2,5% du volume de la solution contenant de la noradrénaline.

5. Procédé selon l'une quelconque des revendications 1 à 4, dans lequel la concentration de noradrénaline dans la solution contenant de la noradrénaline est inférieure à 0,1 mg/ml, de préférence inférieure à 0,06 mg/ml, plus préférablement inférieure à 0,04 mg/ml, en particulier inférieure à 0,025 mg/ml, encore en particulier dans la plage allant de 0,001 à 0,04 mg/ml.

6. Procédé de réduction du nombre de particules subvisibles dans une solution contenant de la noradrénaline, dans lequel la solution de noradrénaline est exempte d'antioxydants, et dans lequel la solution contenant de la noradrénaline est contenue dans un récipient, avec un espace libre, comprenant les étapes suivantes :
a. la dissolution de la noradrénaline et éventuellement d'un ou de plusieurs excipients dans de l'eau désoxygénée ou dégazée, afin de produire une solution contenant de la noradrénaline ;
b. le remplissage de la solution contenant de la noradrénaline dans un récipient, le récipient rempli comprenant la solution contenant de la noradrénaline et un espace libre ;
c. la régulation de la concentration d'oxygène dans l'espace libre à un niveau inférieur ou égal à 1,0 pour cent v/v, de préférence dans la plage allant de 0,15 à 1,0 pour cent v/v, en particulier dans la plage allant de 0,2 à 0,95 pour cent v/v.

7. Produit médicamenteux comprenant :
un récipient hermétiquement scellé comprenant une solution contenant de la noradrénaline et un espace libre,
pouvant être obtenu par un procédé dans lequel la concentration d'oxygène dans la solution contenant de la noradrénaline immédiatement après le scellement du récipient est inférieure à 300 ppb en poids, en particulier inférieure à 100 ppb en poids, et dans lequel la concentration d'oxygène dans l'espace libre immédiatement après le scellement du récipient est inférieure ou égale à 1,0 pour cent v/v,
en particulier pouvant être obtenu par un procédé selon l'une quelconque des revendications 1 à 6, dans lequel la solution de noradrénaline est exempte d'antioxydants,

8. Produit médicamenteux selon la revendication 7, dans lequel la concentration d'oxygène dans la solution contenant de la noradrénaline dans le récipient hermétiquement scellé après équilibrage à 25°C est inférieure à 600 ppb en poids, en particulier inférieure à 340 ppb en poids.

9. Produit médicamenteux selon la revendication 7 ou 8, dans lequel la concentration d'oxygène dans l'espace libre immédiatement après le scellement du récipient se trouve dans la plage allant de 0,15 à 1,0 pour cent v/v, de préférence dans la plage allant de 0,2 à 1,0 pour cent v/v, en particulier dans la plage allant de 0,3 à 0,95 pour cent v/v.

10. Produit médicamenteux selon l'une quelconque des revendications 7 à 9, dans lequel le volume de l'espace libre est inférieur à 11%, de préférence inférieur à 10%, plus préférablement inférieur à 9%, plus préférablement inférieur à 8 % du volume de la solution contenant de la noradrénaline, et éventuellement dans lequel le volume de l'espace libre est d'au moins 1%, de préférence d'au moins 2,5%, du volume de la solution contenant de la noradrénaline,

11. Produit médicamenteux selon l'une quelconque des revendications 7 à 10, dans lequel la concentration de noradrénaline dans la solution contenant de la noradrénaline est inférieure à 0,1 mg/ml, de préférence inférieure à 0,06 mg/ml, plus préférablement inférieure à 0,04 mg/ml, en particulier inférieure à 0,025 mg/ml, encore en particulier dans la plage allant de 0,001 à 0,04 mg/ml.

12. Utilisation d'une atmosphère gazeuse comprenant une concentration d'oxygène inférieure ou égale à 1,0 pour cent v/v, de préférence dans la plage allant de 0,15 à 1,0 pour cent v/v, en particulier dans la plage allant de 0,2 à 0,95 pour cent v/v, comme espace libre dans un récipient comprenant une solution contenant de la noradrénaline, dans laquelle la solution de noradrénaline est exempte d'antioxydants, pour réduire la formation de particules subvisibles dans la solution.

13. Utilisation selon la revendication précédente, dans laquelle la concentration d'oxygène dans la solution contenant de la noradrénaline est inférieure à 600 ppb en poids, en particulier inférieure à 340 ppb en poids, en particulier inférieure à 100 ppb en poids.

14. Produit médicamenteux selon l'une quelconque des revendications 7 à 11, ou produit selon un procédé selon l'une quelconque des revendications 1 à 6, pour une utilisation comme médicament sous forme galénique parentérale, en particulier sous forme galénique parentérale prête à administrer, de préférence dans le traitement du collapsus cardiovasculaire, dans les états de choc associés à de faibles résistances périphériques ou pour rétablir et/ou maintenir des niveaux de pression physiologiques.

15. Procédé selon l'une quelconque des revendications 1 à 6, dans lequel la solution de noradrénaline est exempte d'agents chélateurs.

16. Produit médicamenteux selon l'une quelconque des revendications 7 à 11, dans lequel la solution de noradrénaline est exempte d'agents chélateurs.

17. Utilisation selon la revendication 12 ou 13, dans laquelle la solution de noradrénaline est exempte d'agents chélateurs.
